(19) Europäisches Patentamt
European Patent Office
Office européen des brevets

(11) Veröffentlichungsnummer: **0 299 350 A2**

# (12) EUROPÄISCHE PATENTANMELDUNG

(21) Anmeldenummer: 88110776.7

(22) Anmeldetag: 06.07.88

(51) Int. Cl.4: C07H 15/252 , A61K 31/70

(30) Priorität: 09.07.87 DE 3722698

(43) Veröffentlichungstag der Anmeldung: 18.01.89 Patentblatt 89/03

(84) Benannte Vertragsstaaten: AT BE CH DE ES FR GB GR IT LI LU NL SE

(71) Anmelder: BEHRINGWERKE AKTIENGESELLSCHAFT
Postfach 1140
D-3550 Marburg/Lahn(DE)

(72) Erfinder: Hermentin, Peter, Dr.
Barfüssertor 30
D-3550 Marburg(DE)
Erfinder: Paal, Michael, Dr.
Eppendorfer Landstrasse 6B
D-2000 Hamburg 20(DE)
Erfinder: Kolar, Cenek, Dr.
Deutschhausstrasse 20
D-3550 Marburg(DE)
Erfinder: Kraemer, Hans Peter, Dr.
Birkenweg 16
D-3550 Marburg(DE)
Erfinder: Hoffman, Dieter, Dr.
Im Stetefeld 6
D-3551 Lahntal(DE)
Erfinder: Berscheid, Hans Gerd, Dr.
Rheinlandstrasse 21
D-6231 Schwalbach(DE)
Erfinder: Böttger, Dirk, Dr.
Bahnstrasse 4
D-6237 Liederbach(DE)

(74) Vertreter: Meyer-Dulheuer, Karl-Hermann, Dr. et al
HOECHST Aktiengesellschaft Zentrale Patentabteilung Postfach 80 03 20
D-6230 Frankfurt/Main 80(DE)

## (54) Zytostatisch wirksame Anthracyclinderivate.

(57) Die Erfindung betrifft neue zytostatisch wirksame Anthracyclinderivate der allgemeinen Formel I,

EP 0 299 350 A2

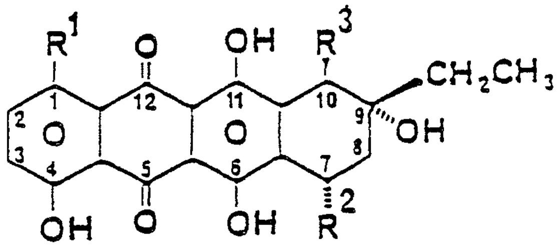

I

in der die Reste $R^1$ Wasserstoff oder eine Hydroxygruppe, $R^2$ Wasserstoff oder eine Hydroxygruppe oder eine Struktur der Formel II und $R^3$ eine Hydroxygruppe oder eine Struktur der Formel II ist,

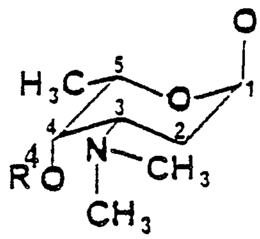

II

worin $R^4$ eine aliphatische oder aromatische Acylgruppe ist, mit der Maßgabe, daß zumindest einer der Reste $R^2$ und $R^3$ eine Struktur nach der Formel II aufweist, welche gegebenenfalls als Salz einer anorganischen oder organischen Säure vorliegen, sowie das Verfahren zur ihrer Herstellung und ihre Verwendung in Arzneimitteln.

## Zytostatisch wirksame Anthracyclinderivate

Die Erfindung bezieht sich auf neue zytostatisch wirksame Anthracyclinderivate und sie betrifft speziell Mono- und Bis-rhodosaminyl-anthracyclinon-Derivate, Verfahren zu ihrer Herstellung sowie ihre Verwendung als Arzneimittel.

Die Substanzklasse der Anthracycline ist schon lange bekannt und seit der Strukturermittlung der Rhodomycine, des Adriamycins bzw. des Daunomycins und dem Bekanntwerden der zytostatischen Wirksamkeit gewisser Vertreter dieser Anthracyclinklasse wurde eine Vielzahl von Anthracyclinen auf biologischem Wege aus Vertretern der Actinomyceten-Gattung Streptomyces isoliert und in ihrer Wirkung erforscht.

Es ist bekannt, daß einige Anthracycline, wie z.B. Adriamycin, Daunomycin, Aclacinomycin, 4′-epi-Adriamycin, 4′-Methoxyadriamycin oder 4′-Desoxyadriamycin bereits für die Therapie von Tumoren verwendet werden.

Bei der tumortherapeutischen Verwendung dieser bekannten Anthracycline liegt ein wesentliches Problem darin, daß sie neben der gewünschten zytostatischen Wirksamkeit unerwünschte Nebenwirkungen aufweisen, wie beispielsweise eine hämatologische oder cardiale Toxizität.

Ausgehend von diesem Stand der Technik ist es Aufgabe der vorliegenden Erfindung, neue, nicht kreuzresistente Anthracyclinderivate zu schaffen, die sich durch ein neues Wirkungsspektrum und eine geringere Toxizität auszeichnen.

Gelöst wird diese erfindungsgemäße Aufgabe mit neuen zytostatisch wirksamen Anthracyclinderivaten, die der nachfolgenden allgemeinen Formel I

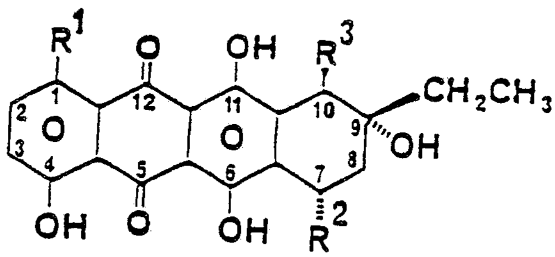

I

entsprechen, welche gegebenenfalls als Salz einer anorganischen oder organischen Säure vorliegen, und in der die Reste folgende Bedeutung haben:

$R^1$ ist Wasserstoff oder eine Hydroxygruppe

$R^2$ ist Wasserstoff oder eine Hydroxygruppe oder eine Struktur der nachfolgenden allgemeinen Formel II,

$R^3$ ist eine Hydroxygruppe oder eine Struktur der nachfolgenden allgemeinen Formel II,

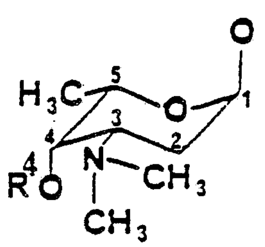

II

worin $R^4$ eine aliphatische oder aromatische Acylgruppe ist, bevorzugt Benzoyl oder substituiertes Benzoyl oder ein aliphatischer Acylrest mit 1-7-Kohlenstoffatomen. Mindestens einer der Reste $R^2$ und $R^3$ ist eine Struktur der Formel II. Wenn beide Reste $R^2$ und $R^3$ Strukturen der Formel II sind, so kann ein $R^4$ auch Wasserstoff sein.

Ist $R^4$ substituiertes Benzoyl, so kann $R^4$ in ortho-, meta- und/oder para-Stellung durch ein oder mehrere Fluor-, Chlor-, Brom- oder Jodatome oder durch Methoxy-, Nitro-, Cyano- oder Azidogruppen substituiert sein. Weitere besonders bevorzugte Anthracyclinderivate ergeben sich aus den Unteransprüchen, die gegebenenfalls auch als Säureadditionssalze von physiologisch unbedenklichen anorganischen oder organischen Säuren vorliegen können.

Anthracycline der Formel I, welche eine oder zwei Strukturen der Formel II mit $R^4$ = H enthalten, sind

zum Teil bereits seit ca. 2 Jahrzehnten bekannt (Brockmann et al., Tetrahedron Letters 1969, 415) und sie können hergestellt werden aus den auf biologischem Wege erhältlichen Anthracyclinverbindungen, die direkt in Form des Rhodosaminderivats vorliegen.

Neuerdings wurde berichtet, daß Anthracycline der Formel I, in welchen $R^1$ Wasserstoff, $R^2$ eine Struktur der Formel II mit $R^4 = H$ und $R^3 = OH$ oder eine Struktur der Formel II mit $R^4 = H$ ist, antitumorale Wirksamkeit besitzen, während eine Struktur der Formel I, in welcher $R^1 = R^2 = H$ und $R^3$ eine Struktur der Formel II mit $R^4 = H$ ist, keine zytostatische Aktivität besitzt (Fleck et al., Studia Biophysica (1984), 104, 215).

Nicht bekannt sind bislang Verbindungen der Formel I, welche eine oder zwei Strukturen der Formel II enthalten, in welchen $R^4$ in mindestens einer Struktur der Formel II eine Acylgruppe ist.

Überraschenderweise wurde nun gefunden, daß sich Verbindungen der Formel I, welche eine oder zwei Strukturen der Formel II mit $R^4 = H$ enthalten, selektiv an der 4′-Hydroxygruppe dieses Rhodosaminyl-Zuckersegments acylieren lassen, ohne daß die anderen in der Verbindung der Formel I vorhandenen alkoholischen oder phenolischen Hydroxygruppen mitacyliert werden.

Ferner wurde gefunden, daß die dabei erhaltenen neuen Anthracyclin-Derivate der Formel I, welche eine oder zwei Strukturen der Formel II enthalten und in welchen $R^1$ bis $R^4$ die genannte Bedeutung haben, zytostatische Aktivität besitzen, ohne daß Kreuzreaktivität zu Adriamycin beobachtet wird.

Ausgehend von diesen Feststellungen ist das erfindungsgemäße Verfahren zur Herstellung der neuen zytostatisch wirksamen Anthracyclinderivate der vorliegenden Erfindung dadurch gekennzeichnet, daß man eine Verbindung der Formel I, in welcher $R^4$ Wasserstoff ist, mit einem reaktionsfähigen Carbonsäurederivat, z.B. dem entsprechenden Säurehalogenid, -anhydrid, -cyanid oder -azid, z.B. der Benzoesäure, einer substituierten Benzoesäure oder einer aliphatischen Carbonsäure, in einem orga nischen Lösungsmittel, wie beispielsweise Chloroform, Dichlormethan oder Dimethylformamid oder einem entsprechenden Lösungsmittelgemisch, in Gegenwart einer Base, wie z.B. Pyridin oder Triäthylamin, bei einer Temperatur zwischen beispielsweise -20° C und +40° C zur korrespondierenden 4′-O-acylierten Verbindung der Formel I umsetzt.

Bevorzugt wird jedoch ein Verfahren, bei welchem die Umsetzung der Ausgangsverbundung in einem Zweiphasensystem erfolgt und zwar in der Weise, daß ein mit Wasser nicht mischbares organisches Lösungsmittel, wie beispielsweise Chloroform oder Dichlormethan, verwendet wird, welches mit einer wäßrigen Phase gerührt wird, in welcher eine geeignete Base, beispielsweise Natriumhydrogencarbonat, enthalten ist, wobei eine Temperatur zwischen beispielsweise 0° C und der Siedetemperatur des organischen Lösungsmittels verwendet werden kann, was einen sehr schonenden Reaktionsablauf und eine Anreicherung des Reaktionsprodukts in der organischen Phase bedingt und einfaches Aufarbeiten erlaubt.

Das reaktionsfähige Carbonsäurederivat wird, sofern die Reaktion im Einphasensystem geführt wird, zweckmäßig in äquivalenter Menge mit der Verbindung gemäß Formel I umgesetzt, da auf diese Weise die Bildung von Nebenprodukten unterdrückt wird und eine Acylierung nur an der 4′-Hydroxygruppe des Rhodosaminyl-Zuckersegmentes stattfindet.

Bevorzugt werden im Rahmen der Erfindung Verbindungen der Formel I, in welcher $R^4$ Benzoyl oder substituiertes Benzoyl ist.

Ebenfalls bevorzugt sind Verbindungen der Formel I, in welchen $R^1 = H$ ist.

Besonders bevorzugt sind insbesondere Verbindungen, in welchen $R^1 = H$ und $R^4$ Benzoyl oder substituiertes Benzoyl ist.

Die nach dem erfindungsgemäßen Verfahren erhaltenen neuen Anthracyclinderivate zeichnen sich durch zytostatische Aktivität aus und können daher zusammen mit den üblichen pharmazeutischen Konfektionierungs- und/oder Verdünnungsmitteln zu Arzneimitteln verarbeitet werden, die in der Krebstherapie Anwendung finden. Die Dosierungs- und Anwendungsweise entspricht dabei im wesentlichen derjenigen für die bekannten Substanzen Adriamycin, Daunomycin, Aclacinomycin, 4′-epi-Adriamycin, 4′-Methoxyadriamycin oder 4′-Desoxyadriamycin.

Die solchermaßen hergestellten Arzneimittel können zusätzlich noch andere Wirkstoffe enthalten, sofern diese zusammen mit den erfindungsgemäßen Verbindungen keine unerwünschten Nebenwirkungen zeigen.

Die zytostatische Wirksamkeit der erfindungsgemäßen Verbindungen wurde anhand von L1210 Leukämiezellen der Maus getestet. Hierzu wurde die Koloniebildung von L1210 Leukämiezellen in Agarplatten herangezogen. Diese Methode dient zum Nachweis des Einflusses der Testsubstanzen auf das Wachstumsverhalten der Zellen über 1 Stunde oder über mehrere Generationen. Bei einer Zellzykluszeit von 10-12 Stunden werden dabei in der Testzeit von 7 Tagen etwa 14 aufeinanderfolgende Generationen beobachtet. Die erfindungsgemäßen zytostatisch wirksamen Substanzen bewirken in diesem Test eine Reduktion der zu beobachtenden Koloniezahl gegenüber einer unbehandelten Kontrollprobe.

Einzelheiten des Testverfahrens ergeben sich aus der nachfolgenden Verfahrensweise zur Ermittlung

der Koloniebildung.

Verfahrensweise zur Ermittlung der Koloniebildung von L1210 Leukämiezellen in Soft-Agar.

500 Leukämiezellen pro Platte wurden mit unterschiedlichen Konzentrationen der Testsubstanz 1 Stunde bei 37 °C inkubiert. Anschließend wurden die Zellen zweimal mit McCoy5A-Medium gewaschen und schließlich in Petrischalen nach Zugabe von 0,3 % Agar ausgegossen. Kontrollen wurden lediglich mit frischem Medium inkubiert. Anstelle der einstündigen Inkubation wurden in manchen Fällen unterschiedliche Konzentrationen und Testsubstanzen der oberen Agarschicht zugemischt, um so eine kontinuierliche Exposition der Zellen über die gesamte Inkubationszeit zu erreichen. Nach Erstarren des Agars wurden die Platten im Brutschrank 7 Tage bei 37 °C inkubiert (5 Vol.-% $CO_2$, 95 % relative Luftfeuchtigkeit). Anschließend wurde die Zahl der entstandenen Kolonien mit einem Durchmesser von mehr als 60 µm gezählt. Die Ergebnisse wurden angegeben als Koloniezahl in behandelten Agarplatten in Prozent der unbehandelten Kontrolle. Aus der so erhaltenen Dosiswirkungskurve wurde die $IC_{50}$ als Maß für die Wirksamkeit der Substanz ermittelt. Die Ergebnisse für die hier beschriebenen Verbindungen im Vergleich zu Adriamycin sind in der nachfolgenden Tabelle 1 zusammengefaßt.

Proliferationstest (MTT-Reduktion)

[ MTT = 3-(4,5-Dimethylthiazol-2-yl)-2,5-diphenyl-tetrazoliumbromid]

L1210, A 549 oder HT 29 in exponentieller Wachstumsphase werden in einer Zelldichte von $5 \times 10^3$ Zellen/ml in RPMI 1640 in einer 96 well Mikrotiterplatte für 72 Stunden mit unterschiedlichen Konzentrationen der Testsubstanz bei 37 °C, 5 % $CO_2$ und 95 % relativer Luftfeuchte inkubiert. Kontrollexperimente erhalten anstelle der Testsubstanz lediglich Wachstumsmedium. Für jede Testsubstanz sowie für die Kontrolle werden 4fach Bestimmungen angesetzt. Nach 65 Stunden Inkubation werden 50 µl einer MTT-Lösung (2,5 mg/ml in Phosphat-gepuffertem Kochsalz) zugegeben. In Gegenwart lebender Zellen wird MTT zu einem dunkelroten unlöslichen Formazanfarbstoff reduziert. Diese Reaktion ist nach 7 Stunden (L1210 Zellen) bzw. nach 24 Stunden (A 549, HT 29 Zellen) beendet und das überstehende Medium wird sorgfältig abgesaugt. Der unlösliche Farbstoff wird durch Zugabe von 100 µl DMSO aufgelöst und die Extinktion der so entstehenden Lösung anschließend für jedes well in einem Multiscan Photometer 340 CC der Fa. Flow bei einer Wellenlänge von 492 nm vermessen.

Aus dem Verhältnis der Extinktionen behandelter und unbehandelter Zellen ergibt sich eine Dosis-Wirkungskurve, aus der die Konzentration, die gerade 50 % der Zellen ($IC_{50}$) abtötet, abgelesen werden kann. Für wiederholte Untersuchungen beträgt der Variationskoeffizient weniger als 15 %.

Kreuzresistenz in vitro

Die Bestimmung der Kreuzresistenz zwischen der jeweiligen Testverbindung und Doxorubicin wird mit Hilfe des MTT-Testes (Methodik siehe vorstehend) an sensitiven bzw. resistenten L1210 Leukämiezellen durchgeführt.

Die resistente Zellinie wurde durch Inkubation einer sensitiven Sublinie mit schrittweise ansteigenden Konzentrationen der Referenzverbindung etabliert.

Die $IC_{50}$ der Testverbindung an der resistenten Sublinie bezogen auf die $IC_{50}$ der sensitiven Sublinie ergibt sowohl den Resistenzgrad für die Testverbindung ($DR_{(T)}$) als auch für die Referenzverbindung ($DR_{(R)}$) entsprechend der Formel

$$DR_{T,R} = \frac{IC_{50} \text{ resistente Zellinie}}{IC_{50} \text{ sensitive Zellinie}}$$

Zusätzlich wird der Grad der Kreuzresistenz (DCR) für die Testverbindung berechnet, entsprechend der

Formel

$$DCR\ \% = \frac{DR_{(T)}^{-1}}{DR_{(R)}} \times 100$$

Im Falle, daß der Wirkungsverlust der Testverbindung an der resistenten Linie im Verhältnis zur sensitiven Linie größer ist als derjenige der Referenzverbindung, ist ein Kreuzresistenzgrad von mehr als 100 % möglich.

Die in Tabelle 1, Teil 3 vorliegenden Ergebnisse zeigen, daß alle Testverbindugen nicht kreuzresistent zu Doxorubicin sind. Die verwendete resistente Zellinie ist selbst 60-80fach resistent für Doxorubicin.

Zur Erläuterung des erfindungsgemäßen Herstellungsverfahrens werden nachfolgend Beispiele 1 bis 24 aufgeführt, in denen bevorzugte erfindungsgemäße Verbindungen nach dem beanspruchten Verfahren hergestellt wurden.

Die Strukturen der hergestellten Verbindungen wurden mittels $^{1}H$- und $^{13}C$-NMR sowie MS-Spektroskopie ermittelt. Die entsprechenden Daten sind in der nachfolgenden Tabelle 2 zusammengestellt. Der Verlauf der Reaktionen sowie die resultierenden Verbindungen wurden dünnschichtchromatographisch oder mit der HPLC-Technik untersucht.

Dünnschichtchromatographie erfolgte auf Kieselgel-Fertigplatten.

Säulenchromatographie erfolgte über Kieselgel 60 vom Durchmesser 20-45 µm oder 0,063-0,200 mm; die Laufmittel-Angaben beziehen sich auf Volumenteile. Die Ausbeuten sind nicht optimiert.

Tabelle 1, Teil 1:

| Verbindungen der Formel I $R^1$ | $R^2$ | $R^3$ | $R^4$ | Addukt | Substanz No. (Beispiel) |
|---|---|---|---|---|---|
| Adriamycin | | | | | |
| H | II | II | Benzoyl | | 1 |
| H | II | II | Benzoyl | HCl | 2 |
| H | II | II | p-Methoxybenzoyl | | 3 |
| H | II | II | p-Methoxybenzoyl | HCl | 4 |
| H | II | II | p-Cyanobenzoyl | | 5 |
| H | II | II | p-Cyanobenzoyl | HCl | 6 |
| H | II | II | p-Nitrobenzoyl | | 7 |
| H | II | II | p-Bromobenzoyl | | 8 |
| H | II | II | Acetyl | | 9 |
| H | II | II | Propionyl | | 10 |
| H | II | II | Monobenzoyl/H | | 11 |
| H | II | II | 4'-(Mono-p-methoxybenzoyl)/4''-H | | 12a |
| H | II | II | 4''-(Mono-p-methoxybenzoyl)/4'-H | | 12b |
| H | II | II | Mono-p-methoxybenzoyl/H | HCl | 13 |
| H | II | II | Mono-p-cyanobenzoyl/H | | 14 |
| H | II | II | Monoacetyl/H | | 15 |
| H | II | II | Monopropionyl/H | | 16 |
| H | II | OH | Benzoyl | | 17 |
| H | II | OH | p-Methoxybenzoyl | | 18 |
| H | II | OH | p-Nitrobenzoyl | | 19 |
| H | II | OH | p-Bromobenzoyl | | 20 |
| H | II | OH | Acetyl | | 21 |
| H | H | II | Benzoyl | | 22 |

Tabelle 1, Teil 2:

| Substanz No. (Beispiel) | Dauerinkubation $IC_{50}$ (µg/ml) | 1h Inkubation $IC_{50}$ (µg/ml) | orientierende Toxizität $LD_{50}$ (mg/kg) 1xip | 3xip, q3d*) |
|---|---|---|---|---|
| Adriamycin | 0,02 | 0,04 | | |
| 1 | - | - | > 100 | |
| 2 | 0,095 | > 1 | | > 85 |
| 3 | 0,115 | > 1 | > 100 | |
| 4 | 0,09 | > 1 | | |
| 5 | 0,105 | > 1 | | |
| 6 | 0,14 | > 1 | | |
| 7 | 2,1 | > 10 | | |
| 8 | 0,3 | > 10 | | |
| 9 | 0,026 | 0,06 | 3.4–6.5 | |
| 10 | 0,12 | 0,25 | | |
| 11 | 0,04 | 0,22 | 10–50 | |
| 12a | 0,0085 | 0,13 | | 10–15 |
| 12b | 0,011 | 0,1 | | 5,33–10,7 |
| 13 | 0,12 | > 1 | | |
| 14 | 0,125 | 0,15 | | |
| 15 | 0,075 | 0,1 | 5–10 | |
| 16 | 0,19 | 0,25 | | |
| 17 | 0,12 | 0,9 | 1–10 | |
| 18 | 0,075 | 0,66 | 10–50 | |
| 19 | 0,03 | 0,55 | > 50 | |
| 20 | - | - | | |
| 21 | 0,08 | 0,7 | 1–10 | |
| 22 | 0,48 | > 1 | 1–10 | |

*) 3xip, q3d: dreimalige Applikation intraperitoneal im Abstand von jeweils drei Tagen

1xip: einmalige Applikation intraperitoneal

Tabelle 1, Teil 3:

| Substanz No. | Test-system | Inkubations-dauer | Resistenz-grad der Zelle | Kreuzresi-stenz zu Adriamycin |
|---|---|---|---|---|
| 1 | MTT | 3d | 1,0 | 0 % |
| 9 | MTT | 3d | 6,0 | 6,0 % |
| 12a | MTT | 3d | 0,75 | 1,3 % |
| 12b | MTT | 3d | 5,0 | 5,0 % |
| 13 | MTT | 3d | 2,5 | 2,5 % |
| 21 | MTT | 3d | 2,2 | 2,4 % |
| Adriamycin (Referenz) | MTT | 3d | 60-80 | 100,0 % |

BEISPIELE

Beispiel 1:

7,10-0-Bis-(4′-O-benzoyl-α-L-rhodosaminyl)-β-rhodomycinon

(Verbindung 1)

700 mg (1 mmol) 7,10-O-Bis-(α-L-rhodosaminyl)-β-rhodomycinon ("β-Rhodomycin-II", Brockmann et al., Tetrahedron Letters 1969, 415; ZA Patent 84/5538) wurden in 500 ml Chloroform gelöst und mit 50 ml gesättigter Natriumhydrogencarbonatlösung versetzt. Zu der auf 0 °C abgekühlten Mischung wurden 310 mg (2,2 mmol) Benzoylchlorid, gelöst in 50 ml Chloroform, zugesetzt und bei Raumtemperatur 16 h im

Dunkeln gerührt. Danach wurde die organische Phase abgetrennt, zweimal mit Wasser gewaschen, mit Magnesiumsulfat getrocknet und im Wasserstrahlvakuum eingeengt. Der resultierende Rückstand wurde säulenchromatographisch (90 g Kieselgel; Elutionsmittel: Toluol/Ethanol = 10:1) gereinigt.
Ausbeute: 685 mg = 0,75 mmol (75%)

Beispiel 2:

HCl-Addukt von Verbindung 1

(Verbindung 2)

Verbindung 1 (20 mg, 0,02 mmol) wurde in 3 ml Methanol gelöst, mit 20 ml 0,1 n Salzsäure auf pH 1 eingestellt, das Methanol im Wasserstrahlvakuum entfernt und die Lösung lyophilisiert.
Ausbeute: 21 mg = 0,02 mmol (100%)

Beispiel 3:

7,10-O-Bis-(4′-O-p-methoxybenzoyl-α-L-rhodosaminyl)-β-rhodomycinon

(Verbindung 3)

2,40 g (3,42 mmol) β-Rhodomycin-II wurden in Analogie zu Beispiel 1 mit 1,30 g (7,62 mmol) 4-Anisoylchlorid zur Reaktion gebracht und aufgearbeitet.
Ausbeute: 2,03 g = 2,1 mmol (61 %)

Beispiel 4:

HCl-Addukt von Verbindung 3

(Verbindung 4)

Die Herstellung erfolgte analog Beispiel 2.

Beispiel 5:

7,10-O-Bis-(4′-O-p-cyanobenzoyl-α-L-rhodosaminyl)-β-rhodomycinon

(Verbindung 5)

300 mg (0,43 mmol) β-Rhodomycin-II wurden in Analogie zu Beispiel 1 mit 157 mg (0,95 mmol 4-Cyanobenzoylchlorid zur Reaktion gebracht und aufgearbeitet.
Ausbeute: 297 mg = 0,31 mmol (72 %)

Beispiel 6:

HCl-Addukt von Verbindung 5

(Verbindung 6)

Die Herstellung erfolgte analog Beispiel 2.

Beispiel 7:

7,10-0-Bis-(4′-O-p-nitrobenzoyl-α-L-rhodosaminyl)-β-rhodomycinon

(Verbindung 7)

175 mg (0,25 mmol) β-Rhodomycin-II wurden in Analogie zu Beispiel 1 mit 102 mg (0,55 mmol) 4-Nitrobenzoylchlorid zur Reaktion gebracht und aufgearbeitet.
Ausbeute: 190 mg = 0,19 mmol (76 %)

Beispiel 8:

7,10-O-Bis-(4′-O-p-bromobenzoyl-α-L-rhodosaminyl)-β-rhodomycinon

(Verbindung 8)

210 mg (0,30 mmol) β-Rhodomycin-II wurden in Analogie zu Beispiel 1 mit 145 mg (0,66 mmol) 4-Bromobenzoylchlorid zur Reaktion gebracht und aufgearbeitet.
Ausbeute: 224 mg = 0,21 mmol (70 %)

Beispiel 9:

7,10-O-Bis-(4′-O-acetyl-α-L-rhodosaminyl)-β-rhodomycinon

(Verbindung 9)

700 mg (1,0 mmol) β-Rhodomycin-II wurden in Analogie zu Beispiel 1 mit 175 mg (2,23 mmol) Acetylchlorid zur Reaktion gebracht und aufgearbeitet.
Ausbeute: 637 mg = 0,81 mmol (81 %)

Beispiel 10:

7,10-O-Bis-(4′-O-propionyl-α-L-rhodosaminyl)-β-rhodomycinon

(Verbindung 10)

300 mg (0,43 mmol) β-Rhodomycin-II wurden in Analogie zu Beispiel 1 mit 90 mg (0,97 mmol) Propionylchlorid zur Reaktion gebracht und aufgearbeitet.
Ausbeute: 276 mg = 0,34 mmol (79 %)

Beispiel 11:

Mischung aus 7-O-Bis-(4′-O-Benzoyl-α-L-rhodosaminyl)-10-0-α-L-rhodosaminyl-β-rhodomycinon

(Verbindung 11a)

und

7-O-α-L-Rhodosaminyl-10-O-(4″-O-benzoyl-α-L-rhodosaminyl)-β-rhodomycinon

(Verbindung 11b)

100 mg (0,14 mmol) β-Rhodomycin-II wurden in Analogie zu Beispiel 1 mit 1 equiv. (20 mg = 0,14 mmol) Benzoylchlorid zur Reaktion gebracht. Nach 1 h wurde die organische Phase, wie in Beispiel 1 beschrieben, aufgearbeitet und das Produktgemisch säulenchromatographisch (20 g Kieselgel; Elutionsmittel: Toluol/Methanol 3:1) getrennt. Neben Dibenzoyl-Derivat (Isolat 1) wurden die Verbindungen 11a und 11b als Isomerenmischung im Verhältnis 11a:11b ca. 1:2 isoliert.
Ausbeute: 40 mg = 0,05 mmol (36 %) Isomerengemisch

Beispiel 12:

Mischung aus 7-O-[(4′-O-p-Methoxybenzoyl)-α-L-rhodosaminyl]-10-O-α-L-rhodosaminyl-β-rhodomycinon

(Verbindung 12a)

und

7-O-α-L-Rhodosaminyl-10-O-[4''-O-(p-methoxybenzoyl)-α-L-rhodosaminyl]-β-rhodomycinon

(Verbindung 12b)

210 mg (0,30 mmol) β-Rhodomycin-II wurden in Analogie zu Beispiel 11 mit 1 equiv. (51 mg = 0,3 mmol) 4-Anisoylchlorid zur Reaktion gebracht und aufgearbeitet. Neben Di-(p-methoxybenzoyl)-Derivat (Isolat 1) wurden die Verbindungen 12a und 12b als Isomerengemisch im Verhältnis 12a:12b ca. 2:3 isoliert. Ausbeute: 117 mg = 0,14 mmol (47 %) Isomerengemisch.

Eine Auftrennung des Isomerengemisches in die jeweils reinen Komponenten erfolgte durch Säulenchromatografie mit einem Laufmittel, bestehend aus Chloroform/Methanol/Essigsäure/Wasser im Verhältnis 77/14/7/2 (Volumenprozente). Die reinen Isomere wurden mit Chloroform gegen wäßriges Natriumhydrogencarbonat ausgeschüttelt.

Beispiel 13:

HCl-Addukt des Isomerengemischs der Verbindungen 12a/12b

(Isomerengemisch der Verbindungen 13a/13b)

Die Herstellung erfolgte analog Beispiel 2.

Beispiel 14:

Mischung aus 7-O-[(4'-O-p-Cyanobenzoyl)-α-L-rhodosaminyl]-10-0-α-L-rhodosaminyl-β-rhodomycinon

(Verbindung 14a)

und

7-O-α-L-Rhodosaminyl-10-O-[4''-O-(p-cyanobenzoyl)-α-L-rhodosaminyl]-β-rhodomycinon

(Verbindung 14b)

300 mg (0,43 mmol) β-Rhodomycin-II wurden in Analogie zu Beispiel 11 mit 1 equiv. (71 mg = 0,43 mmol) 4-Cyanobenzoylchlorid zur Reaktion gebracht und aufgearbeitet. Neben Di- (p-cyanobenzoyl)-Derivat (Isolat 1) wurden die Verbindungen 14a und 14b als Isomerengemisch im Verhältnis 14a:14b ca. 1:2 isoliert.
Ausbeute: 161 mg = 0,19 mmol (44 %) Isomerengemisch

Beispiel 15:

Mischung aus 7-O-[(4′-O-Acetyl-α-L-rhodosaminyl)-10-O-α-L-rhodosaminyl-β-rhodomycinon

(Verbindung 15a)

und

7-O-α-L-Rhodosaminyl-10-O-[4″-O-acetyl-α-L-rhodosaminyl)-β-rhodomycinon

(Verbindung 15b)

100 mg (0,14 mmol) β-Rhodomycin-II wurden in Analogie zu Beispiel 11 mit 1 equiv. (11 mg = 0,14 mmol) Acetylchlorid zur Reaktion gebracht und aufgearbeitet. Neben Diacetyl-Derivat (Isolat 1) wurden die Verbindungen 15a und 15b als Isomerengemisch im Verhältnis 15a:15b ca. 1:6 isoliert.
Ausbeute: 40 mg = 0,054 mmol (39 %) Isomerengemisch

Beispiel 16:

Mischung aus 7-O-[(4′-O-Propionyl-α-L-rhodosaminyl]-10-O-α-L-rhodosaminyl-β-rhodomycinon

(Verbindung 16a)

und

7-O-α-L-Rhodosaminyl-10-O-[4″-O-(propionyl-α-L-rhodosaminyl)-β-rhodomycinon

(Verbindung 16b)

100 mg (0,14 mmol) β-Rhodomycin-II wurden in Analogie zu Beispiel 11 mit 1 equiv. (13 mg = 0,14 mmol) Propionylchlorid zur Reaktion gebracht und aufgearbeitet. Neben Dipropionyl-Derivat (Isolat 1) wurden die Verbindungen 16a und 16b als Isomerengemisch im Verhältnis 16a:16b ca. 1:4 isoliert.
Ausbeute: 38 mg = 0,05 mmol (38 %) Isomerengemisch

Beispiel 17:

7-O-[(4'-O-Benzoyl-α-L-rhodosaminyl)-β-rhodomycinon

(Verbindung 17)

544 mg (1 mmol) 7-O-(α-L-Rhodosaminyl)-β-rhodomycinon ("β-Rhodomycin-I", Brockmann et al., Tetrahedron Letters 1969, 415) wurden in einer Lösungsmittelmischung aus 95 ml Dichlormethan und 5 ml Pyridin gelöst. Der auf 0 °C abgekühlten Lösung wurden 131 mg (1 mmol) Benzoylcyanid zugesetzt. Nach 12 h Rühren bei Raumtemperatur wurde die Mischung im Vakuum eingeengt und der Rückstand wurde chromatographisch über 100 g Kieselgel (Elutionsmittel: Toluol/ Ethanol 10:1) gereinigt.
Ausbeute: 520 mg = 0,80 mmol (80 %)

Beispiel 18:

7-O-[(4'-O-Methoxybenzoyl-α-L-rhodosaminyl)-β-rhodomycinon

(Verbindung 18)

300 mg (0,55 mmol) β-Rhodomycin-I wurden in 30 ml Dichlormethan/Pyridin 10:1 gelöst. 106 mg (0,62 mmol) p-Methoxybenzoylchlorid, gelöst in 20 ml Dichlormethan, wurden innerhalb von 0,5 h zugetropft. Nach 3 h wurde die Reaktionsmischung mit 10 ml Methanol versetzt und anschließend im Vakuum eingeengt. Das resultierende Produkt wurde chromatographisch über 80 g Kieselgel (Elutionsmittel: Toluol/Ethanol 10:1) gereinigt.
Ausbeute: 238 mg = 0,35 mmol (64 %)

Beispiel 19:

7-O-[(4'-O-p-Nitrobenzoyl-α-L-rhodosaminyl)-β-rhodomycinon

(Verbindung 19)

Ausgehend von β-Rhodomycin-I und 4-Nitrobenzoylchlorid wurde, wie bei der Herstellung der Verbindung 18 beschrieben, Verbindung 19 hergestellt.

Beispiel 20:

7-O-[(4'-O-p-Bromobenzoyl-α-L-rhodosaminyl)-β-rhodomycinon

(Verbindung 20)

Ausgehend von β-Rhodomycin-I und 4-Bromobenzoylchlorid wurde, wie bei der Herstellung der Verbindung 18 beschrieben, Verbindung 20 hergestellt.

Beispiel 21:

7-O-(4′-O-Acetyl-α-L-rhodosaminyl)-β-rhodomycinon

(Verbindung 21)

544 mg (1 mmol) β-Rhodomycin-I wurden in Analogie zu Beispiel 11 mit 1 equiv. (79 mg = 1 mmol) Acetylchlorid zur Reaktion gebracht und aufgearbeitet. Der nach Entfernung des Lösungsmittels verbliebene Rückstand wurde säulenchromatographisch (100 g Kieselgel; Elutionsmittel:Toluol/Ethanol 10:1) gereinigt.
Ausbeute: 510 mg = 0.87 mmol (87 %)

Beispiel 22:

10-O-(4′-O-Benzoyl-α-L-rhodosaminyl)-γ-rhodomycinon

(Verbindung 22)

264 mg (0,5 mmol) 10-O-(α-L-rhodosaminyl)-γ-rhodomycinon ("Iremycin", Ihn et al., J. Antibiotics (1980), 33, 1457) wurden in Analogie zu Beispiel 11 mit 1 equiv. (70 mg = 0,5mmol) Benzoylchlorid zur Reaktion gebracht und aufgearbeitet. Der nach Entfernung des Lösungsmittels verblie bene Rückstand wurde säulenchromatographisch (50 g Kieselgel; Elutionsmittel: Chloroform/Methanol/Essigsäure/Ameisensäure 13:1:1:0,4) gereinigt.
Ausbeute: 266 mg = 0,41 mmol (82 %)

Beispiel 23:

Alternativherstellung der Verbindungen 15a und 15b

20 mg β-Rhodomycin-II wurden in 15 ml Chloroform gelöst und mit 20 ml gesättigter Natriumhydrogencarbonatlösung versetzt und nach Zusatz von 1 ml Acetanhydrid 1 h bei 53 °C im Dunkeln gerührt. Danach wurde die organische Phase abgetrennt, zur Tr..kne eingeengt, mit Chloroform und einer gesättigten Lösung von Natriumhydrogencarbonat extrahiert und säulenchromatographisch mit Elutionsmittel Toluol/Methanol (3:1) gereinigt.

Beispiel 24:

Alternativherstellung von Verbindung 21:

20 mg β-Rhodomycin-I wurden in Analogie zu Beispiel 23 umgesetzt und aufgearbeitet und säulenchromatographisch mit Elutionsmittel Toluol/Ethanol (10:1) gereinigt.

Tabelle 2, Teil 1:

| Proton / Verbindung | 1 | 3 | 5 | 7 |
|---|---|---|---|---|
| H-1 | 7.91dd | 7.92dd | 7.93dd | 7.88dd |
| H-2 | 7.72dd | 7.73t | 7.75t 1) | 7.72t |
| H-3 | 7.32dd | 7.33dd | 7.35dd | 7.31dd |
| H-7 | 5.22m | 5.22m | 5.22m | 5.19m |
| H-10 | 5.07s | 5.06s | 5.07s | 5.07s |
| $H_3$-14 | 1.15t | 1.14t | 1.14t | 1.14t |
| H-1', 1'' | 5.67s; 5.66s | 5.65bs | 5.65bs | 5.66bs |
| H-3', 3'' | 2.68ddd; 2.55ddd | 2.68ddd; 2.53ddd | 2.68m; 2.54m | 2.93m; 2.76m |
| H-4', 4'' | 5.52bs; 5.49bs | 5.48bs; 5.46bs | 5.52bs; 5.49bs | 5.59bs; 5.56bs |
| H-5', 5'' | 4.28q; 4.13q | 4.25q; 4.11q | 4.28q; 4.14q | 4.32q; 4.17q |
| $H_3$-6', 6'' | 1.23d; 1.18d | 1.21d; 1.16d | 1.22d; 1.18d | 1.23d; 1.19d |
| $N(CH_3)_2$ | 2.21s; 2.20s | 2.21s | 2.22bs | 2.35s |
| OH-4 | 12.13bs | 12.16bs | 12.12bs | 2) |
| OH-6 | 12.92bs | 12.94bs | 12.93bs | 12.89bs |
| OH-11 | 13.79bs | 13.79bs | 13.81bs | 13.78bs |
| OH-9 | 3.70s | 3.70s | 3.61bs | 2) |
| andere | | 3.87s; 3.86s($OCH_3$) | | |

1) überlagert durch Aromaten-Signale   2) nicht bestimmt

Tabelle 2, Teil 2:

| Proton / Verbindung | 8 | 9 | 10 |
|---|---|---|---|
| H-1 | 7.90d | 7.87d | 7.90d |
| H-2 | 7.73dd | 7.71dd | 7.72t |
| H-3 | 7.32d | 7.29d | 7.31d |
| H-7 | 5.20m | 5.13m | 5.16m |
| H-10 | 5.08s | 5.02s | 5.03s |
| $H_3$-14 | 1.16t | 1.10t | 1.3-1.1m 3) |
| H-1', 1'' | 5.65bs | 5.52bs; 5.49bs | 5.54bd; 5.52bd |
| H-3', 3'' | 2.65m; 2.50m | 2.50m; 2.40m | 2.6-2.3m |
| H-4', 4'' | 5.50bs; 5.45bs | 5.25bs; 5.21bs | 5.27bs; 5.24bs |
| H-5', 5'' | 4.28q; 4.10q | 4.18q; 4.02q | 4.16q; 4.02q |
| $H_3$-6', 6'' | 1.20d | 1.20d | 1.3-1.1m 3) |
| $N(CH_3)_2$ | 2.20s | 2.20s | 2.20s |
| OH-4 | 12.10bs | 2) | 12.12bs |
| OH-6 | 12.93bs | 2) | 12.88bs |
| OH-11 | 13.80bs | 2) | 13.73bs |
| OH-9 | 3.71bs | 3.65bs | 3.62bs |
| andere | | | |

2) nicht bestimmt 3) überlagert durch propionyl-$CH_3$

Tabelle 2, Teil 3:

| Proton / Verbindung | 11a | 11b | 12a | 12b |
|---|---|---|---|---|
| H-1 | | 7.91d | 7.91d | 7.91dd |
| H-2 | | 7.73t | 7.72t | 7.72dd |
| H-3 | | 7.33d | 7.32d | 7.32dd |
| H-7 | | 5.18m | 5.20m | 5.17m |
| H-10 | | 5.06s | 5.03s | 5.05s |
| $H_3$-14 | | 1.13t | 1.13t | 1.13t |
| H-1' | | 5.50bs | 5.64bd | 5.50bs |
| H-1" | | 5.65bs | 5.46bd | 5.64bd |
| H-3' | 2.55ddd | | 2.56ddd | 2) |
| H-3" | | 2.70ddd | 2) | 2.68ddd |
| H-4' | | 3.69bs | 5.48bs | 3.70bs |
| H-4" | | 5.50bs | 3.64bs | 5.45bs |
| H-5' | 4.28q | 4.06q | 4.25q | 4.05q |
| H-5" | 3.92q | 4.11q | 3.92q | 4.11q |
| $H_3$-6' | 2) | 1.40d | 1.21d | 1.40d |
| $H_3$-6" | 1.36d | 1.18d | 1.35d | 1.16d |
| $N(CH_3)_2$ | | 2.22s | 2.20s | 2.22s;2.20s |
| OH-4 | | 12.16bs | 12.16bs | 12.15bs |
| OH-6 | | 12.90bs | 12.90bs | 12.89bs |
| OH-11 | 13.73bs | 13.80bs | 13.72bs | 13.79bs |
| OH-9 | | 3.75bs | 3.64 | 3.70bs |
| andere | | | 3.87s ($OCH_3$) 8.05(d,2H), 6.93(d,2H) | 3.85s ($OCH_3$) 8.03(d,2H), 6.91(d,2H):Ph |

Tabelle 2, Teil 4:

| Proton / Verbindung | 14a | 14b | 15b | 16b |
|---|---|---|---|---|
| H-1 | | 7.92dd | 7.91dd | 7.91dd |
| H-2 | | 7.73t[1)] | 7.72dd | 7.72t |
| H-3 | | 7.33dd | 7.33d | 7.32dd |
| H-7 | | 5.18m | 5.17m | 5.16m |
| H-10 | 5.04s | 5.05s | 5.03s | 5.02s |
| $H_3$-14 | | 1.13t | 1.12t | 1.2-1.1m[3)] |
| H-1' | | 5.49bs | 5.49bs | 5.48bs |
| H-1" | | 5.63bd | 5.53d | 5.53d |
| H-3' | 2.51ddd | | 2.48ddd | 2.51ddd |
| H-3" | | 2.60ddd | | |
| H-4' | | 3.69bs | 3.69bs | 3.68bs |
| H-4" | | 5.47bs | 5.23bs | 5.24bs |
| H-5' | 4.28q | 4.06q | 4.03m | 4.03m |
| H-5" | 3.90q | 4.14q | 4.03m | 4.03m |
| $H_3$-6' | 1.21d | 1.40d | 1.40d | 1.39d |
| $H_3$-6" | 1.35d | 1.17d | 1.15d | 1.2-1.1m[3)] |
| $N(CH_3)_2$ | | 2.20s;2.17s | 2.20s;2.19s | 2.20s;2.17s |
| OH-4 | | 12.15bs | 12.16bs | 12.15bs |
| OH-6 | | 12.92bs | 12.89bs | 12.88bs |
| OH-11 | 2) | 13.81bs | 13.76bs | 13.75bs |
| OH-9 | | 3.72s | 3.71s | 3.68bs |
| andere | | | 2.13s (acetyl-$CH_3$) | |

Tabelle 2, Teil 5:

| Proton / Verbindung | 17 | 18 | 19 | 20 | 21 | 22[4)] |
|---|---|---|---|---|---|---|
| H-1 | 7.88dd | 7.79dd | 7.88dd | 7.88dd | 7.85dd | 2) |
| H-2 | 7.73t | 7.67t | 7.75t | 7.73t | 7.70t | 2) |
| H-3 | 7.32dd | 7.25dd | 7.25dd | 7.30dd | 7.28dd | 7.30 |
| H-7 | 5.16m | 6.67m | 5.20m | 5.20m | 5.12m | 2) |
| H-10 | 4.91s | 4.89s | 4.93s | 5.08s | 4.88s | 2) |
| $H_3$-14 | 1.12t | 1.13t | 1.14t | 1.13t | 1.13t | 1.10t |
| H-1' | 5.70bs | 5.57bs | 5.70bs | 5.65bs | 5.54bs | 5.67bs |
| H-3' | 2.95 | 2.84m | 2.81m | 2.65m | 2) | 3.00m |
| H-4' | 5.58bs | 5.53bs | 5.60bs | 5.50bs | 5.25bs | 5.57bs |
| H-5' | 4.33q | 4.30q | 4.35q | 4.28q | 4.19q | 4.22q |
| $H_3$-6' | 1.20d | 1.20d | 1.24d | 1.20d | 1.20d | 1.21d |
| andere | | 3.85s ($OCH_3$) | | | 2.18s (acetyl-$CH_3$) | |

4) 90 MHz-Spektrum

**Ansprüche**

1. Neue zytostatisch wirksame Anthracyclinderivate der allgemeinen Formel I,

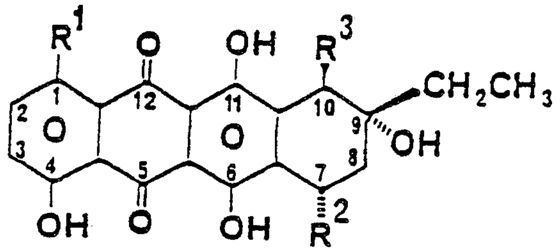

I

in der die Reste folgende Bedeutung haben:
$R^1$ ist Wasserstoff oder eine Hydroxygruppe
$R^2$ ist Wasserstoff oder eine Hydroxygruppe oder eine Struktur der nachfolgenden allgemeinen Formel II,
$R^3$ ist eine Hydroxygruppe oder eine Struktur der nachfolgenden allgemeinen Formel II,

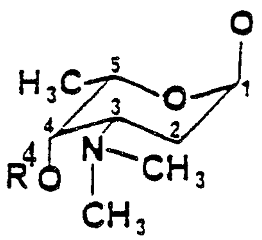

II

worin $R^4$ eine aliphatische oder aromatische Acylgruppe ist, mit der Maßgabe, daß zumindest einer der Reste $R^2$ und $R^3$ eine Struktur nach der Formel II darstellt und wenn beide Reste $R^2$ und $R^3$ eine Struktur der Formel II darstellen, $R^4$ in einem dieser Reste auch Wasserstoff sein kann, und die gegebenenfalls als Salz einer anorganischen oder organischen Säure vorliegen.

2. Anthracyclinderivate nach Anspruch 1,
**dadurch gekennzeichnet,**
daß $R^4$ Benzoyl oder in ortho-, meta-, und/oder para-Stellung durch ein oder mehrere Fluor-, Chlor-, Brom- oder Jodatome oder durch Methoxy-, Nitro-, Cyano- oder Azidogruppen substituiertes Benzoyl oder ein aliphatischer Acylrest mit 1-7 Kohlenstoffatomen ist.

3. Anthracyclinderivate nach Anspruch 1,
**dadurch gekennzeichnet,**
daß $R^1$ Wasserstoff ist, $R^2$ und $R^3$ jeweils eine Struktur der Formel II darstellen, in der $R^4$ in beiden Resten $R^2$ und $R^3$ die gleiche Acylgruppe ist.

4. Anthracyclinderivate nach Anspruch 1,
**dadurch gekennzeichnet,**
daß $R^1$ Wasserstoff ist und $R^2$ und $R^3$ jeweils eine Struktur der Formel II darstellen, wobei in der einen Struktur der Formel II $R^4$ Wasserstoff ist und in der anderen Struktur der Formel II $R^4$ eine aliphatische oder aromatische Acylgruppe ist, wobei die paarweise korrespondierenden Verbindungen auch jeweils in einer Mischung vorliegen können.

5. Anthracyclinderivate nach Anspruch 1,
**dadurch gekennzeichnet,**
daß $R^1$ Wasserstoff, $R^2$ eine Struktur der Formel II ist, in welcher $R^4$ die angegebene Bedeutung hat und $R^3$ eine Hydroxygruppe ist.

6. Anthracyclinderivate nach Anspruch 1,
**dadurch gekennzeichnet,**
daß $R^1$ Wasserstoff, $R^2$ Wasserstoff oder eine Hydroxygruppe und $R^3$ eine Struktur der Formel II ist, in welcher $R^4$ die angegebene Bedeutung hat.

7. Anthracyclinderivate nach Anspruch 1,
**dadurch gekennzeichnet,**
daß $R^1$ eine Hydroxygruppe ist, $R^2$ und $R^3$ jeweils eine Struktur der Formel II darstellen, in der $R^4$ in beiden Resten $R^2$ und $R^3$ die gleiche Acylgruppe ist.

8. Anthracyclinderivate nach Anspruch 1,
**dadurch gekennzeichnet,**
daß $R^1$ eine Hydroxygruppe ist und $R^2$ und $R^3$ jeweils eine Struktur der Formel II darstellen, wobei in der einen Struktur der Formel II $R^4$ Wasserstoff ist und in der anderen Struktur der Formel II $R^4$ eine aliphatische oder aromatische Acylgruppe ist, wobei die paarweise korrespondierenden Verbindungen auch jeweils in einer Mischung vorliegen können.

9. Anthracyclinderivate nach Anspruch 1,
**dadurch gekennzeichnet,**
daß $R^1$ eine Hydroxygruppe, $R^2$ eine Struktur der Formel II ist, in welcher $R^4$ die angegebene Bedeutung hat und $R^3$ eine Hydroxygruppe darstellt.

10. Anthracyclinderivate nach Anspruch 1,
**dadurch gekennzeichnet,**
daß $R^1$ eine Hydroxygruppe, $R^2$ Wasserstoff oder eine Hydroxygruppe und $R^3$ eine Struktur der Formel II ist, in welcher $R^4$ die angegebene Bedeutung hat.

11. Verfahren zur Herstellung von Anthracyclinderivaten nach Anspruch 1,
**dadurch gekennzeichnet,**
daß man eine Verbindung der Formel I, in welcher $R^4$ Wasserstoff ist, mit einem reaktionsfähigen Carbonsäurederivat in einem organischen Lösungsmittel oder einem entsprechenden Lösungsmittelgemisch, in Gegenwart einer Base, bei einer Temperatur zwischen -20°C und +40°C zu einer Verbindung umsetzt, in welcher $R^4$ die angegebene Bedeutung hat.

12. Verfahren zur Herstellung von Anthracyclinderivaten nach Anspruch 1,
**dadurch gekennzeichnet,**
daß man eine Verbindung der Formel I, in welcher $R^4$ Wasserstoff ist, mit einem reaktionsfähigen Carbonsäurederivat in einem Zweiphasensystem, bestehend aus einem mit Wasser nicht mischbaren organischen Lösungsmittel und einer wäßrigen Phase, in welcher eine geeignete Base enthalten ist, umsetzt.

13. Verfahren nach Anspruch 11,
**dadurch gekennzeichnet,**
daß die Verbindung der Formel I mit einer äquivalenten Menge des reaktionsfähigen Carbonsäurederivates pro zu substituierender Zuckereinheit umgesetzt wird.

14. Verwendung eines Anthracyclinderivates nasch Anspruch 1 in einem Arzneimittel.

15. Arzneimittel, enthaltend ein Anthracyclinderivat nach Anspruch 1.

Patentansprüche für folgende Vertragsstaaten: ES und GR

1. Verfahren zur Herstellung neuer zytostatisch wirksamer Anthracyclinderivate der allgemeinen Formel I,

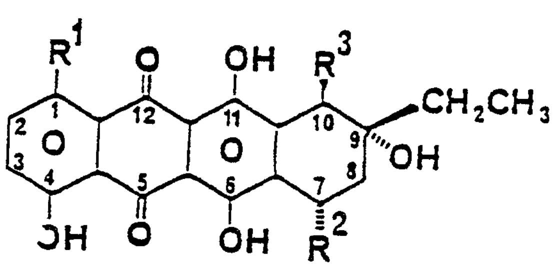

I

in der die Reste folgende Bedeutung haben:
$R^1$ ist Wasserstoff oder eine Hydroxygruppe
$R^2$ ist Wasserstoff oder eine Hydroxygruppe oder eine Struktur der nachfolgenden allgemeinen Formel II,
$R^3$ ist eine Hydroxygruppe oder eine Struktur der nachfolgenden allgemeinen Formel II,

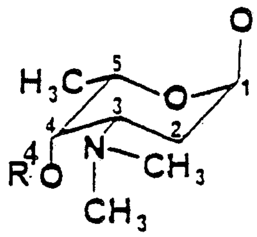

II

worin $R^4$ eine aliphatische oder aromatische Acylgruppe ist, mit der Maßgabe, daß zumindest einer der Reste $R^2$ und $R^3$ eine Struktur nach der Formel II darstellt und wenn beide Reste $R^2$ und $R^3$ eine Struktur der Formel II darstellen, $R^4$ in einem dieser Reste auch Wasserstoff sein kann, und die gegebenenfalls als Salz einer anorganischen oder organischen Säure vorliegen,

**dadurch gekennzeichnet,**

daß man eine Verbindung der Formel I, in welcher $R^4$ Wasserstoff ist, mit einem reaktionsfähigen Carbonsäurederivat in einem organischen Lösungsmittel oder einem entsprechenden Lösungsmittelgemisch, in Gegenwart einer Base, bei einer Temperatur zwischen -20°C und +40°C zu einer Verbindung umsetzt, in welcher $R^4$ die angegebene Bedeutung hat.

2. Verfahren zur Herstellung von Anthracyclinderivaten nach Anspruch 1,

**dadurch gekennzeichnet,**

daß man eine Verbindung der Formel I, in welcher $R^4$ Wasserstoff ist, mit einem reaktionsfähigen Carbonsäurederivat in einem Zweiphasensystem, bestehend aus einem mit Wasser nicht mischbaren organischen Lösungsmittel und einer wäßrigen Phase, in welcher eine geeignete Base enthalten ist, umsetzt.

3. Verfahren nach Anspruch 1,

**dadurch gekennzeichnet,**

daß die Verbindung der Formel I mit einer äquivalenten Menge des reaktionsfähigen Carbonsäurederivates pro zu substituierender Zuckereinheit umgesetzt wird.

4. Verfahren zur Herstellung eines Arzneimittels unter Verwendung eines Anthracyclinderivates, hergestellt nach Anspruch 1.